# EUROPEAN PATENT APPLICATION

(11) **EP 4 462 195 A1**
(43) Date of publication of application: **13.11.2024**
(21) Application number: 24173709.7
(22) Date of filing: 02.05.2024
(51) Int. Cl.: G05B 9/02, G05B 15/02

(54) **AIR QUALITY MONITORING**

(30) Priority: 04.05.2023 FI 20235498
(71) Applicant: Helvar Oy Ab, 02150 Espoo (FI)
(72) Inventor: JUSLÉN, Henri, 02150 ESPOO (FI); TAKALA, Pasi, 02150 ESPOO (FI); PAUL, Måns, 02150 ESPOO (FI)
(74) Representative: Berggren Oy

(57) **Abstract**

According to an example embodiment, an apparatus (110-k, 150, 160) for monitoring air quality in a space is provided, the apparatus comprising: a control portion (112-k, 152, 162) arranged to: obtain sensor data that is indirectly descriptive of a concentration of a predefined substance in air within said space, apply at least one prediction model to predict, based on occurrences of one or more predefined patterns in said sensor data that are associated with respective changes in the concentration of the predefined substance in the air within said space, timing of the concentration of the predefined substance in the air within said space reaching a predefined threshold level due to one of one or more processes that result in introduction of said predefined substance into the air within said space, and issue, based on the predicted timing, at least one indication that pertains to applicability of said space for human occupants.

## Description

### TECHNICAL FIELD

The example and non-limiting embodiments of the present invention relate to monitoring of air quality in a space.

### BACKGROUND

Ultraviolet germicidal irradiation (UVGI) is a commonly applied method for disinfecting indoor spaces. UVGI relies on usage of ultraviolet C (UV-C) irradiation for inactivating various micro-organisms in a space, including bacteria and viruses such as SARS-CoV-2. While UVGI has been traditionally applied for purification in medical facilities and laboratory environments, recently it has found use in air purification in more generic contexts, including homes and office spaces.

UVGI may be provided, for example, via usage of UVGI device(s) such as UV-C LED lamps installed in a space in a permanent or semi-permanent manner to enable conveniently carrying out air purification in the space when needed. Since UVGI relies on the UV-C irradiation breaking molecular bonds of the virus and bacteria, relatively high levels of UV-C irradiation are also harmful for humans (and for other living things) and entry to the space upon operation of the UVGI device(s) is typically prevented. In addition to direct harmful effect arising from the UV-C irradiation, application of UVGI may also result in increase in concentrations various indoor air pollutants in the space, such as ozone, volatile organic compounds (VoCs) and/or particulate matter such as PM_{2.5} that are likewise harmful for humans when inhaled.

While disinfection via usage of UVGI serves here as an example of a process that results in introducing various pollutants to a space that may be harmful to humans residing in the space, similar effect may arise from various other processes as well, e.g. from operation of ozonizer applied for air purification in the space or e.g. from chemicals applied in cleaning operations carried out in the space.

### SUMMARY

It is an object of the present invention to provide a technique for observing changes in indoor air quality in a space in view of its safety for human occupants.

According to an example embodiment, an apparatus for monitoring air quality in a space is provided, the apparatus comprising: a control portion arranged to: obtain sensor data that is indirectly descriptive of a concentration of a predefined substance in air within said space, apply at least one prediction model to predict, based on occurrences of one or more predefined patterns in said sensor data that are associated with respective changes in the concentration of the predefined substance in the air within said space, timing of the concentration of the predefined substance in the air within said space reaching a predefined threshold level due to one of one or more processes that result in introduction of said predefined substance into the air within said space, and issue, based on the predicted timing, at least one indication that pertains to applicability of said space for human occupants.

According to another example embodiment, an apparatus for deriving at least one prediction model for predicting timing of a concentration of a predefined substance in air within a space reaching a predefined threshold level is provided, the apparatus comprising a learning portion arranged to: obtain history data comprising a history of sensor data that is at least indirectly descriptive of the concentration of the predefined substance in the air within said space for a learning period and a history of concentrations of said predefined substance in the air within said space for the learning period; and carry out a learning procedure to derive, based on the history data, at least one prediction model that is applicable for predicting, based on occurrences of one or more patterns in the sensor data that are associated with respective changes in the concentration of the predefined substance in the air within said space, timing of the concentration of the predefined substance in the air within said space reaching the predefined threshold level due to the one of the one or more processes that result in introduction of the predefined substance into the air within said space.

According to another example embodiment, a method for monitoring air quality in a space is provided, the method comprising: obtaining sensor data that is at least indirectly descriptive of a concentration of a predefined substance in air within said space; applying at least one prediction model to predict, based on occurrences of one or more predefined patterns in said sensor data that are associated with respective changes in the concentration of the predefined substance in the air within said space, timing of the concentration of the predefined substance in the air within said space reaching a predefined threshold level due to one of one or more processes that result in introduction of said predefined substance into the air within said space, and issuing, based on the predicted timing, at least one indication that pertains to applicability of said space for human occupants.

According to another example embodiment, a method for deriving at least one prediction model for predicting timing of a concentration of a predefined substance in air within a space reaching a predefined threshold level is provided, the method comprising: obtaining history data comprising a history of sensor data that is at least indirectly descriptive of the concentration of the predefined substance in the air within said space for a learning period and a history of concentrations of said predefined substance in the air within said space for the learning period; and carrying out a learning procedure to derive, based on the history data, at least one prediction model that is applicable for predicting, based on occurrences of one or more patterns in the sensor data that are associated with respective changes in the concentration of the predefined substance in the air within said space, timing of the concentration of the predefined substance in the air within said space reaching the predefined threshold level due to the one of the one or more processes that result in introduction of the predefined substance into the air within said space.

The exemplifying embodiments of the invention presented in this patent application are not to be interpreted to pose limitations to the applicability of the appended claims. The verb "to comprise" and its derivatives are used in this patent application as an open limitation that does not exclude the existence of also unrecited features. The features described hereinafter are mutually freely combinable unless explicitly stated otherwise.

Some features of the invention are set forth in the appended claims. Aspects of the invention, however, both as to its construction and its method of operation, together with additional objects and advantages thereof, will be best understood from the following description of some example embodiments when read in connection with the accompanying drawings.

### BRIEF DESCRIPTION OF FIGURES

The embodiments of the invention are illustrated by way of example, and not by way of limitation, in the figures of the accompanying drawings, where
Figure 1 illustrates a block diagram of some logical components of a system according to an example;
Figure 2 illustrates a block diagram of some components of a sensor apparatus according to an example;
Figure 3 illustrates a method according to an example;
Figure 4 illustrates a method according to an example;
Figure 5 illustrates a block diagram of some components of a lighting system gateway according to an example;
Figure 6 illustrates a block diagram of some components of a lighting system server according to an example; and
Figure 7 illustrates a block diagram of some components of an apparatus according to an example.

### DESCRIPTION OF SOME EMBODIMENTS

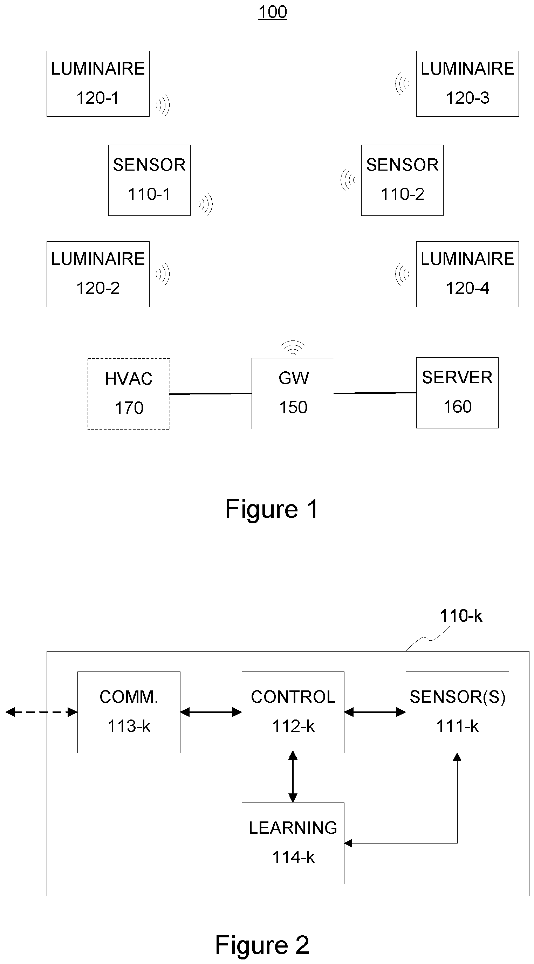

Figure 1 illustrates a block diagram of some elements of a lighting system 100 according to an example, where lighting system 100 may be arranged for illuminating a space. The lighting system 100 as illustrated in Figure 1 includes luminaires 120-1, 120-2, 120-3,120-4 and sensor apparatuses 110-1, 110-2. The luminaires 120-1 to 120-4 represent one or more luminaires 120, where an individual luminaire may be referred to via a reference number 120-j. The sensor apparatuses 110-1 and 110-2 represent one or more sensor apparatuses 110, where an individual sensor apparatus may be referred to as a sensor apparatus 110-k. The one or more luminaires 120 may be considered as a lighting sub-system (within the lighting system 100) and the one or more sensor apparatuses may be considered as a monitoring sub-system (within the lighting system 100).

Each of the luminaires 110-j may be disposed in a respective location of the space and arranged to illuminate a respective location of the space. Each of the sensor apparatuses 110-k may be disposed in a respective location of the space and it may comprise respective one or more sensors arranged to monitor respective one or more environmental characteristics in the respective location of the space. The respective portion of the space monitored by sensor(s) of the sensor apparatus 110-k may be also referred to as a respective volume of the space. The space under consideration may comprise an indoor space, which may be a closed space such as a room in a building or a certain area an open space within a building. The one or more sensor apparatuses 110 and the one or more luminaires 120 may be communicatively coupled to each other via a communication network. Consequently, the one or more sensor apparatuses 110 and the one or more luminaires 120 may be considered as respective nodes of a lighting control network. While the illustration of Figure 1 suggests wireless communication between the nodes of the lighting control network, in another example at least part of the lighting control network may be provided via a wired communication medium.

As an example of operation of the light system 100 according to the example of Figure 1, the one or more sensor apparatuses 110 may be arranged to supply sensor data captured therein over the lighting control network to the one or more luminaires 120 to facilitate each of the luminaires 120-k controlling its respective light outputs at least partially based on sensor data received from the one or more sensor apparatuses 110. In another example, the one or more sensor apparatuses 110 may be arranged to derive lighting control commands based on the sensor data captured therein and submit the lighting control commands over the lighting control network to the one or more luminaires 120 for each of the luminaires 120-k controlling its respective light outputs at least partially based on the lighting control commands received from the one or more sensor apparatuses 110.

The lighting system 100 may further comprise or it may be communicatively coupled to one or more control apparatuses that may be enable occupants of the space to control at least some aspects of the light output provided via the one or more luminaires 120 and/or that may be applicable for providing the occupants with information concerning operation of the lighting system 100. In this regard, the one or more control apparatuses may comprise a control panel including one or more switches, one or more buttons, one or more knobs, a touch panel, a touchscreen, etc. for enabling the occupants to enter control information for controlling the light output and/or a display, a touchscreen or one or more visual indicators of other kind for providing the occupants with information that pertains operational status of the lighting system 100 and/or environmental conditions in the space illuminated by the lighting system 100. Additionally or alternatively, the control apparatuses may comprise mobile devices communicatively coupled to the lighting control network e.g. via the lighting control gateway 150 or via another node of the lighting control network.

According to an example, as shown in the illustration of Figure 1, the lighting system 100 may further comprise a lighting system gateway 150, which is also provided as a node of the lighting control network. The lighting control gateway 150 may be communicatively coupled to a lighting system server 160, thereby providing a communicative coupling between the lighting control network and the lighting system server 160. The lighting system server 160 may be applicable for receiving and storing at least part of the sensor data captured in the one or more sensor apparatuses 110, providing further aspects of lighting control e.g. based on the sensor data received thereat and/or based on external lighting control information supplied to the lighting system server 160, carrying out analysis of the sensor data and/or other computational tasks to support operation of the lighting system 100, etc. Alternatively or additionally, the lighting system 100 may be communicatively coupled to or provided as part of a building automation system that may also serve to provide other services or functionalities available in the space illuminated via operation of the lighting system 100 and/or in the building including the space illuminated via operation of the lighting system 100. As an example in this regard, the lighting system gateway 150 (as shown in the illustration of Figure 1) and/or the lighting system server 160 may be communicatively coupled to a HVAC system controller 170 and/or to a building automation controller of other kind, thereby communicatively coupling the lighting system 100 to one or more entities of the building automation system. According to another example, the lighting system 100 may be a stand-alone one in the sense that it is not coupled to other systems in the space and/or in the building and it may be hance configured for autonomous or semi-autonomous operation in terms of illuminating the space under consideration.

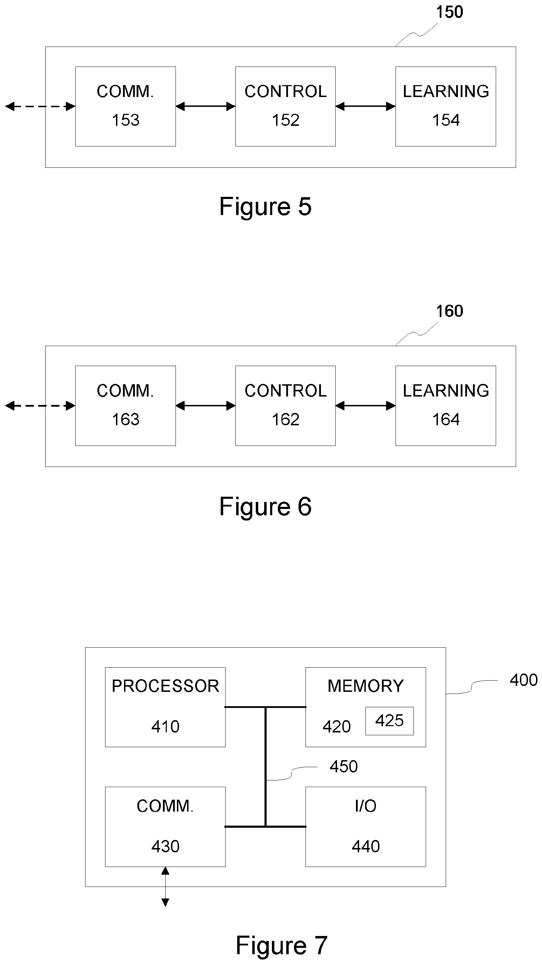

The lighting control gateway 150 may be implemented via operation of a computer apparatus that comprises one or more processors and one or more memories, where the one or more memories are arranged to store computer program code that, when executed by the one or more processors, implements the operation of the lighting control gateway 110. The lighting control server 160 may be implemented via operation of one or more computer apparatuses that each comprises respective one or more processors and respective one or more memories, where the respective one or more memories of the one or more computer apparatuses are arranged to store respective portions of computer program code that, when executed by the respective one or more processors, implement the operation of the lighting control server 160. In this regard, the one or more computer apparatuses may be arranged to provide a cloud computing service. More detailed examples of using a computer apparatus to implement the lighting control gateway 150 and/or at least a portion of the lighting control server 160 are described later in this text with references to Figure 7.

Figure 2 illustrates a block diagram of some elements of a sensor apparatus 110-k according to an example, comprising a sensor portion 111-k, a control portion 112-k, a communication portion 113-k and a learning portion 114-k. The sensor portion 111-k comprises one or more sensors, each arranged to capture sensor data that is descriptive of a respective environmental characteristic at the location monitored by the sensor apparatus 110-k, the control portion 112-k is arranged to control various aspects of operation of the sensor apparatus 110-k and to process the sensor data captured via operation of the sensor portion 111-k, and the communication portion 113-k enables wireless (and/or wired) communication with other entities e.g. via the lighting control network. The learning portion 114-k may be arranged to configure at least one aspect of operation of the control portion 112-k in terms of processing the sensor data therein in the course of operation of the sensor apparatus 110-k via carrying out learning procedure (which is described in the following in further detail).

The sensor portion 111-k may comprise or it may be implemented as a sensor module. Each of the one or more sensors of the sensor portion 111-k may be arranged to supply the control portion 112-k with a respective sensor signal that is descriptive of the respective environmental characteristics at the location monitored by the sensor apparatus 110-k. In this regard, the one or more sensors of the sensor portion 111-k may be communicatively coupled, e.g. via respective electrical wires, to the control portion 112-k. The one or more sensors of the sensor portion 111-k may comprise respective sensors of different type. Non-limiting examples of sensors that may be included in the sensor portion 111-k include the following:
- a motion sensor for monitoring occupancy at the location monitored by the sensor apparatus 110-k, e.g. a passive infrared (PIR) sensor, a microwave radar, a lidar, a video camera, a thermal camera, etc.;
- a light sensor for measuring ambient light level at the location monitored by the sensor apparatus 110-k, e.g. photodetector such as photodiode;
- a temperature sensor for measuring ambient temperature at the location monitored by the sensor apparatus 110-k;
- a humidity sensor for measuring air humidity at the location monitored by the sensor apparatus 110-k;
- a sound sensor (e.g. a microphone or a microphone array) for capturing sounds at the location monitored by the sensor apparatus 110-k;
- a carbon dioxide (CO₂) sensor for measuring concentration of CO₂ at the location monitored by the sensor apparatus 110-k;
- a volatile organic compound (VOC) sensor for measuring total concentration of VOCs (tVOC) at the location monitored by the sensor apparatus 110-k.

Herein, references to the sensor data being descriptive of the respective environmental characteristic *at the location monitored by* the sensor apparatus 110-k is to be construed broadly, encompassing respective environmental characteristic in a portion of the space or in a volume of the space at the monitored location of the space and in its vicinity.

The control portion 112-k may be implemented via operation of a computer apparatus included in the sensor apparatus 110-k. According to an example, the computer apparatus may comprise one or more processors and one or more memories, where the one or more memories are arranged to store computer program code that, when executed by the one or more processors, implements the operation of the control portion 112-k according to the present disclosure. More detailed examples of using a computer apparatus to implement the control portion 112-k are described later in this text with references to Figure 7.

The control portion 112-k may be arranged to record and/or derive respective sensor indications based on respective sensor signals received from the sensor portion 111-k, where the sensor indications recorded and/or derived at the control portion 112-k may be referred to as respective local sensor indications since they are based on sensor signals captured locally at the sensor apparatus 110-k. The local sensor indications recorded and/or derived at the control portion 112-k may be referred to as sensor indications captured at the sensor apparatus 110-k and they may be jointly referred to as local sensor data, whereas the (local) sensor indications of the (local) sensor data may be alternatively referred to as (local) sensor values. As non-limiting examples in this regard (in view of the exemplifying sensor types described above), the operation of the control portion 112-k include the following:
- Deriving local occupancy state indications based on a sensor signal received from a motion sensor provided in the sensor portion 111-k, where in various examples the local occupancy state indication may indicate e.g. one of occupancy or non-occupancy at the location monitored by the sensor apparatus 110-k, a likelihood of occupancy at the location monitored by the sensor apparatus 110-k, or a measure that is at least indirectly descriptive of a number of occupants at the location monitored by the sensor apparatus 110-k.
- Deriving local tVOC level indications based on a sensor signal received from a tVOC sensor provided in the sensor portion 111-k, where the tVOC level indications may indicate e.g. total concentration at the location monitored by the sensor apparatus 110-k.
- Deriving sound level indications based on a sensor signal received from a sound sensor provided in the sensor portion 111-k, where the sound level indications may indicate e.g. sound pressure level captured at the sound sensor.

The control portion 112-k may store at least part of the local sensor indications into the memory provided in the sensor apparatus 110-k for subsequent use. In this regard, the respective local sensor indications captured at the sensor apparatus 110-k and stored in the memory therein constitute a respective time series of local sensor indications, which is indicative of the respective environmental characteristic in the monitored location of the space as a function time. The captured local sensor indications may be applied, for example, for lighting control within the lighting system 100 and/or for analysis of one or more aspects of environmental conditions at the location monitored by the sensor apparatus 110-k. As an example of lighting control that at least partially relies on the local sensor indications, the local occupancy state indications derived at the sensor apparatus 110-k may be applied for controlling the light output of the one or more luminaires 120 in dependence of the occupancy detected in the space such that the space is illuminated when occupancy is detected and the space is not illuminated when no occupancy is detected.

Along the lined described in the foregoing, the sensor apparatus 111-k may enable communicative coupling e.g. with other nodes of the lighting control network using wireless or wired communication means. In this regard, in case wireless communication means are applied, the communication portion 113-k may comprise a wireless transceiver that is capable of communicating with respective wireless transceivers in other apparatuses, e.g. in other nodes of the lighting control network. The wireless communication may be provided by using a suitable short-range wireless communication technique known in the art that enables communication over ranges from a few meters up to a few hundred meters. Examples of suitable wireless communication techniques include Bluetooth, Bluetooth Low-Energy (LE), ZigBee, WLAN/Wi-Fi according to an IEEE 802.11 family of standards, etc. Further examples include infrared communications and other non-radio-based short-range communication techniques. The choice of the communication technique and network topology for a specific implementation of the lighting arrangement 100 may depend e.g. on the required communication range and/or requirements with respect to energy-efficiency of the communication means. As a concrete non-limiting example, the wireless communication may rely on a wireless mesh network model, for example on a mesh network according to the Bluetooth or Bluetooth LE Mesh networking protocol known in the art. In case wired communication means are applied, the communication in this regard may be provided via a wired bus using a lighting control protocol, such as the Digital Addressable Lighting Interface (DALI) specified in a series of technical standards IEC 62386, whereas the communication portion 113-k may provide a communication interface arranged for communication according to the applicable lighting control protocol. Further examples of providing wired communication with other apparatuses include Power over Ethernet (PoE) and packet switched communication networks such as Internet Protocol (IP) based networks.

As pointed out in the foregoing, the one or more sensor apparatuses 110 may be installed for operation in an indoor space, whereas the local sensor data obtained via operation of the respective sensor portions of 111-k of the one or more sensor apparatuses 110 may be descriptive of various environmental characteristics of said space. In this regard, respective sensor data originating from the sensors of certain type may be at least indirectly descriptive of air quality in the space and, consequently, the local sensor indications captured at the sensor apparatus 110-k may be appliable for estimating and/or predicting one or more aspects of indoor air quality (IAQ) in the space in which the respective sensor apparatus 110-k is being operated. In particular, the air in the space may become contaminated by excessive levels of one or more substances due to operation of one of a one or more processes when they are carried out within and/or in close proximity of the space. In this regard, the respective concentrations of the one or more substances in the air within the space typically starts to increase upon a beginning (or an activation) of such a process while their respective concentrations typically start to decrease upon an ending (or a deactivation) of the respective process. In this regard, excessive concentration of any substance in the air may make it harmful to a human occupant, whereas certain known indoor air pollutants may be problematic already at relatively low concentrations. Examples of commonly appearing indoor air pollutants include (secondhand) tobacco smoke, radon, various types of molds, carbon monoxide, carbon dioxide, various VOCs, ozone, particulate matter of various sizes, etc. In this regard, the indoor air pollutants may be also referred to as (indoor) air quality deteriorators or as (indoor) air impurities.

A particular example of such a process involves the UVGI described in the foregoing, which is useful in providing air purification in the space via the UV-C radiation breaking molecular bonds of the virus and bacteria, even relatively low level of continuous UV-C irradiation (that may be harmless to human occupants as such) has been found out to result in increase in concentrations of various substances to respective levels that render them as indoor air pollutants in the space. Non-limiting examples in this regard include substances such as ozone, certain volatile organic compounds (VoCs) and/or particulate matter such as PM_{2.5} that are potentially harmful for humans when inhaled. While UVGI serves as a concrete example of a process that may be applied substantially regularly and that may result in introducing certain harmful substances into the air within the space, the same or similar substances may be, additionally or alternatively, introduced into the space via processes of other kind, such as via operation of ozonizer applied for air purification in the space or via cleaning operations carried out in the space due to chemical applied for cleaning and disinfection. Even though such processes may result in introducing a plurality of potentially harmful substances into the air within the space, in the following various aspects of making use of the local sensor data available at the sensor apparatus 110-k for monitoring air quality in the space are described with references to monitoring a concentration of a predefined indoor air pollutant in the space - whereas these examples readily generalize into monitoring of a concentration of any (potentially harmful) substance in the air within the space and/or simultaneous monitoring of respective concentrations of a plurality of predefined substances in the air (e.g. indoor air pollutants) in the space, *mutatis mutandis.*

In view of the above, the control portion 112-k may be arranged to make use of the local sensor data available therein to estimate and/or predict changes in the indoor air quality in the location of the space monitored by the sensor apparatus 110-k. In this regard, the estimation and/or prediction may be carried out based on a portion of the local sensor data captured therein, e.g. based on respective time portions the respective time series local sensor indications stored in the memory at the sensor apparatus 110-k. In this regard, in some example only part of the sensors of the sensor portion 111-k may provide respective sensor data that is applicable for monitoring the concentration of the predefined indoor air pollutant in the space and, consequently, only the respective sensor data (e.g. the respective time series of sensor indications) originating from such sensors may be applied for monitoring of the concentration of the predefined indoor air pollutant in the space. Nevertheless, the following examples predominantly refer to the (local) sensor data in general in the interest of brevity and clarity of the description.

In the framework of the Figure 1 the sensor apparatus 110-k primarily serves as an element that captures sensor data that is applicable for lighting control in the space under consideration, whereas the concentration of the predefined indoor air pollutant is typically not directly relevant for the lighting control. Consequently, even though the sensor portion 111-k may include sensors that serve to capture sensor data this is indirectly relevant for the light control, it may still not be meaningful to provide the sensor portion 111-k of the sensor apparatus 110-k with a (typically highly specialized) sensor that is designed for directly monitoring the concentration of the predefined indoor air pollutant and/or it may not be meaningful to continuously apply such a sensor e.g. due to its power consumption. On the other hand, some of the sensor data that is directly or indirectly relevant for the lighting control is typically also indirectly descriptive of changes in the concentration of the predefined indoor air pollutant in the space over time. Further in this regard, in some examples, some sensor of the sensors portion 111-k may be applied for capturing respective sensor data only when presence of one or more occupants in the space is detected (e.g. via operation of the motion sensor provided in the sensor portion 111-k) in order to reduce power consumption of the sensor portion 111-k during time periods of non-occupancy in the space.

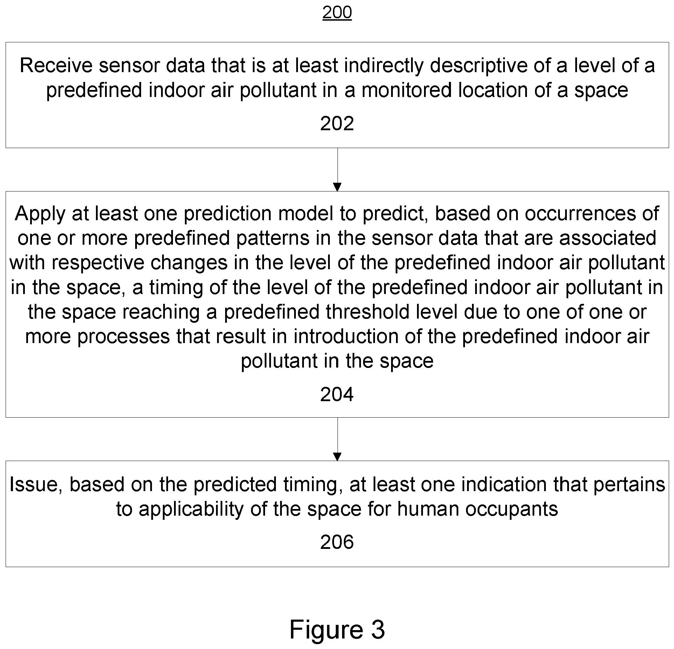

Therefore, instead of using the sensor data available at the control portion 112-k for directly estimating or predicting an instantaneous amount of the predefined indoor air pollutant in the space, the control portion 112-k may be arranged to observe the sensor indications captured at the sensor apparatus 110-k in order to estimate or predict upcoming changes in the concentration of the predefined indoor air pollutant in view of the space becoming inapplicable or applicable for human occupants at a future time, thereby allowing for coordinated manner of controlling access to the space. In a particular example, the indoor air quality in the space may be provided via carrying out a method 200 illustrated in Figure 3, via operation of the control portion 112-k, where the method 200 may comprise the following steps:
- receive sensor data that is at least indirectly descriptive of the concentration of the predefined indoor air pollutant in the monitored location of the space (block 202);
- apply at least one prediction model to predict, based on occurrences of one or more predefined patterns in the sensor data that are associated with respective changes in the concentration of the predefined indoor air pollutant in the space, a timing of the concentration of the predefined indoor air pollutant in the space reaching a predefined threshold level due to one of one or more processes that result in introduction of the predefined indoor air pollutant in the space (block 204), and
- issue, based on the predicted timing, at least one indication that pertains to applicability of the space for human occupants (block 206).

Referring to the sensor data under consideration (cf. block 202), the sensor at least indirectly descriptive of the concentration of the predefined indoor air pollutant in the monitored location of the space e.g. in one of the following ways:
- the sensor data under consideration may include a time series of sensor indications that is directly descriptive of the concentration of the predefined indoor air pollutant, e.g. sensor indications recorded and/or derived based on a sensor signal originating from a sensor that serves to measure the concentration of the predefined air pollutant;
- the sensor data under consideration may include a time series of sensor indications that is indirectly descriptive of the concentration of the predefined indoor air pollutant, e.g. sensor indications recorded and/or derived based on a sensor signal originating from a sensor that serves to measure an environmental characteristics different from the concentration of the predefined air pollutant but that has a value that changes in dependence and/or together with the concentration of the predefined air pollutant in a manner defined in the at least one prediction model;
- the sensor data under consideration may include a first time series of sensor indications that is directly descriptive of the concentration of the predefined indoor air pollutant (as described above) and a second time series of sensor indications that is indirectly descriptive of the concentration of the predefined indoor air pollutant (as described above).

Referring now to the prediction (cf. block 204), the prediction may proceed from a first scenario where the concentration of the predefined indoor pollutant is below the predefined threshold level and the prediction may hence involve predicting the timing of the concentration of the predefined indoor pollutant increasing to a level that exceeds the predefined threshold level, whereas in another example the prediction may proceed from a second scenario where the concentration of the predefined indoor pollutant is above the predefined threshold level and the prediction may hence involve predicting the timing of the concentration of the predefined indoor pollutant falling to a level that is below the predefined threshold level. In this regard, concentrations of the predefined indoor air pollutant above the predefined threshold level may be considered unacceptable for human occupants while concentrations of the predefined indoor air pollutant below the predefined threshold level may be considered acceptable for human occupants.

The one or more predefined patterns in the sensor data may comprise one or more predefined onset patterns and/or one or more predefined ending patterns. In this regard, the one or more predefined onset patterns may be considered in the first scenario where the concentration of the predefined indoor pollutant is below the predefined threshold level and each onset pattern may be associated with a beginning of a respective one of the one or more processes that result in introduction of the predefined indoor air pollutant in the space. In this regard, the at least one prediction model may comprise a first prediction model that is applicable for predicting a first time period that is indicative of the time it takes from detection of an occurrence of a certain one of the one or more predefined onset patterns until the concentration of the predefined indoor air pollutant in the space exceeds the predefined threshold level. Hence, the control portion 112-k may apply the first prediction model to detect an occurrence of one the one or more predefined onset patterns and to predict the duration of the first time period between the occurrence time of the respective predefined onset pattern and the time at which the concentration of the predefined indoor air pollutant exceeds the predefined threshold level.

Along similar lines, the one or more predefined ending patterns may be considered in the second scenario where the concentration of the predefined indoor pollutant is above the predefined threshold level and each ending pattern may be associated with an ending of a respective one of the one or more processes that result in introduction of the predefined indoor air pollutant in the space. In this regard, the at least one prediction model may comprise a second prediction model that is applicable for predicting a second time period that is indicative of the time it takes from detection of an occurrence of a certain one of the one or more predefined ending patterns until the concentration of the predefined indoor air pollutant in the space falls below the predefined threshold level. Hence, the control portion 112-k may apply the second prediction model to detect an occurrence of one the one or more predefined ending patterns and to predict the duration of the second time period between the occurrence time of the respective predefined ending pattern and the time at which the concentration of the predefined indoor air pollutant falls below the predefined threshold level.

In other words, the one or more predefined patterns in the sensor data may comprise one or more predefined onset patterns and/or one or more predefined ending patterns, each associated, respectively, with a beginning or an ending of a respective one of the one or more processes that result in introduction of the predefined indoor air pollutant. Moreover, the at least one prediction model comprises at least one prediction model that is applicable for predicting a time period that is indicative of time from detecting an occurrence of one of said predefined onset patterns or ending patterns until the concentration of the predefined indoor air pollutant within the space reaching the predefined threshold level (i.e. either rising above the threshold level or falling below the threshold level).

The one or more predefined patterns in the sensor data available at the control portion 112-k considered in the prediction (cf. block 204) may be defined via respective changes (or lack thereof) in sensor indications captured at the sensor apparatus 110-k over a monitoring period of predefined duration. In this regard, each of the predefined patterns in sensor data may define a respective change pattern for one or more time series of sensor indications over the monitoring period of a predefined duration. Consequently, an occurrence of a certain predefined pattern in the sensor data may be detected via the control portion 112-k monitoring the respective one or more time series of sensor indications captured therein in the course of operation of the sensor apparatus 110-k and identifying presence of the respective change patterns defined in the respective predefined pattern in the one or more time series of sensor indications under consideration. As an example in this regard, a duration of the monitoring period (and hence the temporal `length' of the predefined pattern) may be in a range from a few seconds to a few hours.

According to an example, a certain predefined pattern may define a change pattern for a predefined single time series of sensor indications, where the change pattern may involve an increasing trend or a decreasing trend in the respective sensor indication value, e.g. one of the following:
- a change from a first value that is below a predefined threshold to a second value that is above the predefined threshold;
- a change from a first value that is above a predefined threshold to a second value that is below the predefined threshold;
- an increase (over the monitoring period) that is larger than a predefined threshold;
- a decrease (over the monitoring period) that is larger than a predefined threshold.

According to another example, a certain predefined pattern may define respective change patterns for two or more time series of sensor indications, where each of the two or more change patterns may include one of the change patterns outlined above. As examples in this regard, the certain predefined pattern may involve a first change pattern for a first time series of sensor indications and a second change pattern for a second time series of sensor indications, where the first and second change patterns may both involve an increasing trend, the first and second change patterns may both involve a decreasing trend, or one of the first and second change patterns may include an increasing trend and the other one may include a decreasing trend.

In a further example, a certain predefined pattern may define respective change patterns for two or more time series of sensor indications, where one of the two or more change patterns may include one of the change patterns outlined above and another one of the two or more change patterns may define e.g. one of the following:
- a value that remains substantially stationary over the monitoring period;
- a value that remains above a predefined threshold over the monitoring period;
- a value that remains below a predefined threshold over the monitoring period.

In a further example, a certain predefined pattern may define a respective change pattern that involves both increase and decrease of the underlying sensor indication value according to a predefined curve. In this regard, the certain change pattern may only include this kind of change pattern for a single time series of sensor values, or the certain change pattern may include a first change pattern of this type and a second change pattern according one of the change pattern types described above.

In various examples, the one or more time series of sensor indications considered in the one or more predefined patterns may be the same for each of the one or more predefined patterns or the one or more time series of sensor indications considered in the one or more predefined patterns may vary from one predefined pattern to another. In non-limiting examples, the one or more time series of sensor indications considered via the one or more predefined patterns may comprise the time series of tVOC indications and/or the time series of occupancy indications, whereas in other examples the one or more time series of sensor indications considered in the one or more predefined patterns may, alternatively or additionally, comprise respective time series of one or more other sensor indications captured at the sensor apparatus 110-k (e.g. ones recorded or captured based on respective sensor signals originating from the exemplifying sensor types described in the foregoing).

Referring back to characteristics of the at least one prediction model applied by the control portion 112-k, an applicable prediction model, e.g. the first prediction model and/or the second prediction model described in the foregoing, may comprise one of the following:
- an artificial neural network, ANN, arranged to determine the timing of the concentration of the predefined indoor air pollutant in the space reaching the predefined threshold level based on occurrences of said one or more predefined patterns, or
- a statistical model arranged to determine the timing of the concentration of the predefined indoor air pollutant in the space changing between unacceptable and acceptable levels based on occurrences of said one or more predefined patterns.

In this regard, the ANN serving as the prediction model may comprise, for example, a convolutional neural network (CNN), a recurrent neural network (RNN) or a logic learning machine (LLM), and it may be trained to recognize patterns in the sensor data captured at the sensor apparatus and to carry out the prediction based a history of sensor data captured in the space under consideration or in a space of similar characteristics (e.g. in terms of its size and typical use) during a learning period in view of information of measured concentrations of the predefined indoor air pollutant during the learning period and possibly further in view of a knowledge of respective timings of beginnings and endings of the one or more processes that result in introduction of the predefined indoor air pollutant in the space during the learning period. The respective histories of the sensor data and the measured concentrations of the predefined indoor air pollutant, possibly complemented by the history of the respective timings of the beginnings and endings of the one or more processed under consideration may be jointly referred to as learning data. The statistical model may comprise, for example, a linear regression model, a non-linear regression model or a polynomial regression model derived based on the learning data via usage of a suitable estimation technique known in the art, whereas in other examples a mathematical model of other kind with its parameters derived based on the learning data may be employed.

In various examples, the at least one prediction model to be applied by the control portion 112-k may be derived via a learning procedure carried out by the sensor apparatus 110-k (as suggested via the example of Figure 2) or by another apparatus. In this regard, the other apparatus may comprise basically any apparatus provided with the history data required for determination of the at least one prediction model, e.g. the lighting system gateway 150, the lighting system server 160, an entity of the building automation system network, etc.

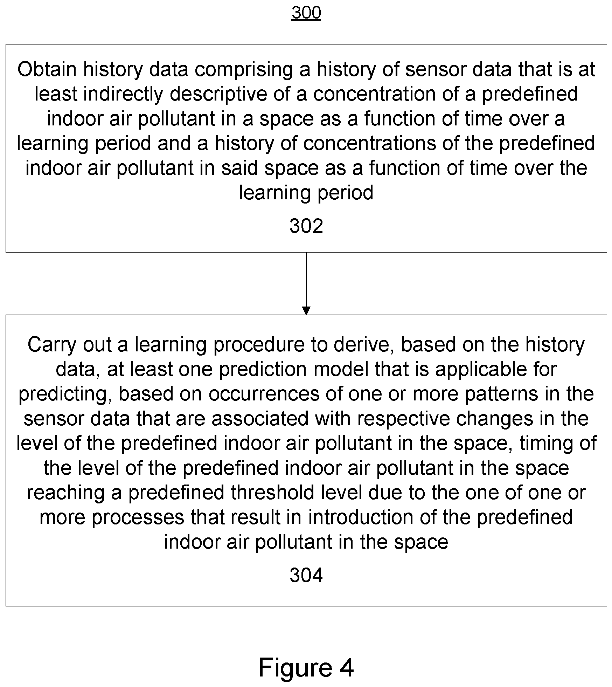

The at least one prediction model may be derived e.g. according to a method 300 illustrated in Figure 4. The method 300 may be carried out, for example, by the learning portion 114-k and it may comprise the following steps:
- obtain history data comprising a history of sensor data that is at least indirectly descriptive of the concentration of the predefined indoor air pollutant in the space under consideration as a function of time over a learning period and a history of concentrations of the predefined indoor air pollutant in the space under consideration as a function of time over the learning period (block 302);
- carry out the learning procedure to derive, based on the history data, at least one prediction model that is applicable for predicting, based on occurrences of one or more patterns in the sensor data that are associated with respective changes in the concentration of the predefined indoor air pollutant in said space, timing of the concentration of the predefined indoor air pollutant in the space reaching the predefined threshold level due to the one of the one or more processes that result in introduction of the predefined indoor air pollutant in the space (block 304).

Hence, the respective histories of the sensor data captured in the space under consideration and the concentrations of the predefined indoor air pollutant in said space serve as the history data applied as the learning data, where the history data covers the learning period. In an example where the at least one prediction model comprises at least one ANN, the learning procedure may involve supervised learning where the history of sensor data serves as input data to the at least one ANN, where the at least one ANN is trained to predict the timing of the concentration of the predefined indoor air pollutant reaching the predefined threshold level via using the history of the concentrations of the predefined indoor air pollutant as the reference data (i.e. the 'ground truth' data). In this regard, the learning procedure may rely on the at least one ANN learning to identify one or more onset patterns in the sensor data that temporally coincide with a scenario where the concentration of the predefined indoor air pollutant starts to increase (e.g. from a baseline concentration) and hence indicate a beginning of a process that results in introducing the predefined indoor air pollutant in the space under consideration. Additionally or alternatively, the learning procedure may rely on the at least one ANN learning to identify one or more ending patterns in the sensor data that temporally coincide with a scenario where the concentration of the predefined indoor air pollutant starts to decrease (e.g. from a local maximum) and hence indicate an ending of a process that results in introducing the predefined indoor air pollutant in the space under consideration.

The learning procedure may comprise training the first ANN to learn to identify occurrences of one or more onset patterns in the sensor data that indicate respective beginnings of the one or more processes under consideration and to predict the respective timings from the respective beginnings of the one or more processes to the concentration of the predefined indoor air pollutant increasing to a level that is above the predefined threshold level. Additionally or alternatively, the learning procedure may comprise training the second ANN to learn to identify occurrences of one or more ending patterns in the sensor data that indicate respective endings of the one or more processes under consideration and to predict the respective timings from the respective endings of the one or more processes to the concentration of the predefined indoor air pollutant decreasing to a level that is below the predefined threshold level.

In some examples, the history data serving as the learning may further include the respective histories of the timings of the beginnings (e.g. activations) and the endings (e.g. deactivations) of the one or more processes that result in introduction of the predefined indoor air pollutant in the space under consideration during the learning period. In such examples, the learning procedure may consider the respective timings of the beginning and endings of the processes under consideration as further reference data that serves as the 'ground truth' data with respect to actual beginnings and endings of the one or more processes under consideration

In the scenario where the learning procedure to derive the at least one prediction model is carried out via operation of the learning portion 114-k of the sensor apparatus 110-k, as an example, the history of the sensor data may be readily captured and stored in the memory of the sensor apparatus 110-k prior to the learning procedure based on the sensor signals received from the sensor portion 111-k. In other examples, the history data may be captured using another sensor apparatus operated in the space or in a space of substantially similar characteristics or the history data may be obtained via simulations or experiments carried out in the space or in another space of substantially similar characteristics. According to an example, the history of the concentrations of the predefined indoor air pollutant in the space under consideration may be obtained from a further sensor coupled to the sensor apparatus 110-k, which further sensor is arranged to directly monitor the concentration of the predefined indoor air pollutant, whereas the further sensor may be coupled to the sensor apparatus 110-k or it may be activated only for the duration of the learning period. Alternatively, the history of the concentrations of the predefined indoor air pollutant in the space under consideration for the learning period may be captured using an apparatus separate from sensor apparatus 110-k and the captured history of the concentrations of the predefined indoor air pollutant in said space may be transferred to the sensor apparatus 110-k for application in derivation of the at least one prediction model.

In case the learning procedure to derive the at least one prediction model further makes use of the respective histories of the timings of the beginnings (e.g. activations) and/or the endings (e.g. deactivations) of the one or more processes that result in introduction of the predefined indoor air pollutant in the space under consideration, according to an example, another apparatus implementing one of said processes may be communicatively coupled to the sensor apparatus 110-k and the sensor apparatus 110-k may receive respective control signals related to an activation (e.g. a beginning) of the respective process and/or to a deactivation (e.g. an ending) of the respective process and record their timings for subsequent use in the learning procedure carried out by the learning portion 114-k. In another example, the respective histories of the respective timings of the beginnings (e.g. activations) and/or the endings (e.g. deactivations) of the one or more processes under consideration may be captured at another apparatus and transferred to the sensor apparatus 110-k for subsequent use in the learning procedure carried out by the learning portion 114-k. In a further example, the respective histories of the respective timings of the beginnings (e.g. activations) and/or the endings (e.g. deactivations) of the one or more processes under consideration may be received at the sensor apparatus 110-k as user input entered via a user interface of the sensor apparatus 110-k.

Referring back to the control portion 112-k issuing the at least one indication that pertains to the applicability of the space under consideration for human occupants (cf. block 206), the control portion 112-k may issue an indication regarding non-applicability of the space for human occupants in response to detecting one of the one or more predefined onset patterns and/or issue an indication regarding applicability of the space in response to detecting one of the one or more ending patterns after detection of a corresponding onset pattern. In various examples, one or more of the following may apply:
- an indication of non-applicability may indicate the predicted timing of the space becoming non-applicable for human occupants;
- an indication of non-applicability of the space may indicate current non-applicability of the space for human occupants;
- an indication of applicability may indicate the predicted timing of the space becoming applicable for human occupants;
- an indication of applicability of the space may indicate current applicability of the space for human occupants.

According to an example, the respective indication of applicability or non-applicability of the space for human occupants may comprise a signal provided to another apparatus, which may adjust its operation accordingly, whereas in another example the respective indication of applicability or non-applicability of the space may comprise a visual indication and/or an audible indication provided in the space and/or in its immediate vicinity. As an example of a visual indication, the control portion 112-k may control one or more display devices that are communicatively coupled thereto to display a text and/or a symbol that is descriptive of the applicability or non-applicability of the space, where the text and/or symbol may indicate the current applicability or non-applicability of the space and/or it may indicate the timing of the space becoming applicable or non-applicable. The one or more display devices may be arranged within the space and/or at entry points (e.g. beside one or more doors leading) to the space, e.g. together with or as part of a control panel provided for controlling lighting in the space. In another example, the one or more display devices may comprise mobile devices communicatively coupled to the sensor unit 110-k either directly or via another entity of the lighting control network. An audible indication may involve an audible indication of current (or upcoming) non-applicability of the space, e.g. via rendering a predefined alarm sound within the space and/or in its immediate vicinity.

As another example of a visual indication, the control portion 112-k may control respective light outputs of at least one of the one or more luminaires 120 in a specific manner to indicate one of applicability of non-applicability of the space e.g. by providing 'normal' lighting to indicate applicability and by varying the light intensity of the light output of at least one of the luminaires 120-k between a 'normal' light intensity and a lower light intensity (e.g. blinking the light output) at a frequency that is perceivable by a human observer to indicate non-applicability. In this regard, the frequency of light intensity variation may be provided at a predefined frequency throughout the period of non-applicability of the space, whereas in another example the light intensity variation may be provided at a frequency that reflects the estimated amount of the predefined indoor air pollutant in the space.

Additionally or alternatively, the control portion 112-k may further apply the at least one prediction model to identify a process that is identified as a source of the predefined indoor air pollutant in the space and issue an indication that pertains to the identified process. In this regard, as described in the foregoing, each of the one or more predefined patterns in the sensor data are associated with one of the one or the one or more processes that may serve as the source of the predefined indoor air pollutant and, consequently, identification of an occurrence of a certain one of the one or more predefined patterns also identifies the associated one of the one or more processes. Consequently, the control portion 112-k may carry out e.g. the following:
- apply the at least one prediction model to identify, based on a detected occurrence of one of said one or more predefined patterns in the sensor data, one of the one or more processes that result in introduction of the predefined air pollutant in the space, and
- issue at least one indication that pertains to the identified one of the one or more processes.

In this regard, the indication may comprise a visual indication displayed via the one or more display devices, where the visual indication includes an identification (e.g. text and/or symbol) that identifies the process that serves as the source of the predefined indoor air pollutant. In a further example, the indication that pertains to the identified one of the one or more processes (and the visual indication thereof) may further indicate a beginning of the identified one of the one or more processes or an ending of the identified one of the one or more processes. In this regard, a beginning of the respective process may be identified via an occurrence of the onset pattern associated with the beginning of the respective process and an ending of the respective process may be identified via an occurrence of the ending pattern associated with the respective process.

The examples provided in the foregoing at least implicitly describe derivation of the at least one prediction model via the learning procedure carried out by the learning portion 114-k of the sensor apparatus 110-k and application of the at least one prediction model by the control portion 112-k of the sensor apparatus 110-k. Other examples in this regard include the following:
- The at least one prediction model may be derived at another apparatus and transferred to the sensor apparatus 110-k for subsequent use by the control portion 112-k therein.
- The at least one prediction model may be derived (by the learning portion 114-k) at the sensor apparatus 110-k and transferred to another apparatus for subsequent use therein.
- The at least one prediction model may be derived and applied at another apparatus.

In various examples in this regard, the other apparatus may comprise a sensor portion similar to the sensor portion 111-k of the sensor apparatus 110-k and use this sensor portion to capture the sensor data applied in the learning procedure and/or for carrying out the prediction via usage of the at least one prediction model after its derivation, whereas in other examples the other apparatus may receive the sensor data applied in the learning procedure and/or for carrying out the prediction via usage of the at least one prediction model after its derivation from the sensor apparatus 110-k. The other apparatus may receive the other pieces of history data applied in the learning procedure as described in the foregoing for the learning procedure carried out by the learning portion 114-k, *mutatis mutandis.* In case the learning procedure is carried out in another apparatus, the learning portion 114-k of the sensor apparatus 110-k may be omitted.

The other apparatus carrying out the derivation of the at least one prediction model may comprise any computing apparatus provided with sufficient computational resources. As a non-limiting example in this regard, the other apparatus arranged to carry out the learning procedure and/or to apply the at least one prediction model comprises the lighting system gateway 150, which is illustrated in Figure 5 with a control portion 152, a communication portion 153 and a learning portion 154. The control portion 152 may be arranged to control various aspects of operation of the lighting system gateway 150 and the communication portion 153 may be arranged for wireless and/or wired communication with other entities. In case the lighting system gateway 150 is applied for derivation of the at least one prediction model, the learning portion 154 may be arranged to carry out the learning procedure to derive the at least one prediction model based on the history of sensor data received and/or captured at the lighting system gateway 150. In case the lighting system gateway 150 is applied for application of the at least one prediction model, the control portion 152 may be arranged to apply the at least one prediction model based on the sensor data received and/or captured at the lighting system gateway 150.

In another example, the other apparatus arranged to carry out the learning procedure and/or to apply the at least one prediction model comprises the lighting system server 160, which is illustrated in Figure 6 with a control portion 162, a communication portion 163 and a learning portion 164. The control portion 162 may be arranged to control various aspects of operation of the lighting system server 160 and the communication portion 163 may be arranged for (wireless and/or) wired communication with other entities. In case the lighting system server 160 is applied for derivation of the at least one prediction model, the learning portion 164 may be arranged to carry out the learning procedure to derive the at least one prediction model based on the history of sensor data received at the lighting system server 160. In case the lighting system server 160 is applied for application of the at least one prediction model, the control portion 162 may be arranged to apply the at least one prediction model based on the sensor data received at the lighting system server 160.

As described in the foregoing, each of the sensor apparatuses 110-k may be disposed in a respective location of the space to monitor respective one or more environmental characteristics in the respective location of the space. Consequently, in a scenario where two or more sensor apparatuses 110-k are applied, each sensor apparatus 110-k may monitor a respective location within the space and issue respective indications that pertain to applicability of the space for human occupants at the respective location of the space. In a variant of such an approach, the two or more sensor apparatuses 110-k may be arranged for operation in different spaces of a building, the respective indications that pertain to applicability of the space for human occupants pertain to different spaces of the building. The respective control portions 112-k in each of the two or more sensor apparatuses 110-k may transmit the respective indications derived therein to an external apparatus, e.g. to the HVAC system controller 170 or a building automation control apparatus of other kind, which hence obtains information regarding the applicability or non-applicability of various locations of the space and/or the building for human occupants. In another approach, the respective control portions 112-k in each of the two or more sensor apparatuses 110-k may transmit the respective indications derived therein to the lighting system gateway 150, which may process and/or analyze the received indications and transmit the result of the processing / analysis to the HVAC system controller 170, whereas in a variant of this another approach the lighting system gateway 150 may forward the indications received from the two or more sensor apparatuses 110-k to the lighting system server 160 for processing / analysis therein and for transmission of the result of the processing / analysis to the HVAC system controller 170 or a building automation control apparatus of other kind.

Referring to a scenario where the building automation system controller, such as the HVAC system controller 170, receives the respective indication that pertain to the applicability of the respective location of the space or the respective space of the building from the one or more sensor apparatuses 110, the building automation system controller may control environmental conditions in the location or space under consideration in consideration of the predicted timing of the location or space becoming applicable or non-applicable for human occupants. In an example where the building automation controller comprises the HVAC system controller 170, it may adjust an aspect of HVAC operation in consideration of the respective indications received from the one or more sensor apparatuses 110. As an example in this regard, the HVAC system controller 170 may obtain from a certain sensor apparatus 110-k an indication that suggests non-applicability of the respective location or space until a certain moment of time while on the other hand the HVAC system controller 170 may obtain indication (e.g. via occupancy prediction or from a room reservation system) regarding an expected occupancy in the respective location or space during a period its non-applicability for human occupants. Consequently, the HVAC system controller 170 may respond to such a situation by controlling an aspect of HVAC operation in a manner that facilitates making the location or space applicable for human occupants faster than using the default setting of the HVAC for said aspect, e.g. via adjusting the air conditioning or ventilation in the location or space for faster circulation of air in order to facilitate prompt reduction of the concentration of the predefined indoor air pollutant in the location of space under consideration.

The examples provided in the foregoing are at least implicitly described in the framework of the lighting system 100 illustrated via the example of Figure 1, where the one or more sensor apparatuses 110 are applied to provide sensor data that is applicable for controlling at least some aspects of the light output from the one or more luminaires 120 of the lighting system 100. Application of the one or more sensor apparatuses 110 as part of the lighting system 100 is, however, a non-limiting example, whereas in other examples the one or more sensor apparatuses 110 may be applied to provide a monitoring system that is separate and/or independent of the lighting system 100, the sensor apparatuses 110-k serving as respective nodes of a sensor network. In such an approach the indication of the applicability of the space for human occupants may be provided means other than the one or more luminaires 120 or the monitoring system may be provided with a luminaire or a light output device for the purpose of providing the indication(s).

Figure 7 illustrates a block diagram of some components of an apparatus 400 that may be employed to implement operations described with references to the control portion 112-k, 152, 162 and/or the learning portion 114-k, 154, 164. The apparatus 400 comprises a processor 410 and a memory 420. The memory 420 may store data and computer program code 425. The apparatus 400 may further comprise communication means 430 for wired or wireless communication with other apparatuses and/or user I/O (input/output) components 440 that may be arranged, together with the processor 410 and a portion of the computer program code 425, to provide the user interface for receiving input from a user and/or providing output to the user. In particular, the communication means 430 may comprise the communication portion 113-k, 153, 163 and the user I/O components may include user input means, such as one or more keys or buttons, a keyboard, a touchscreen or a touchpad, etc. The user I/O components may include output means, such as a display or a touchscreen. The components of the apparatus 400 are communicatively coupled to each other via a bus 450 that enables transfer of data and control information between the components.

The memory 420 and a portion of the computer program code 425 stored therein may be further arranged, with the processor 410, to cause the apparatus 400 to perform at least some aspects of operation of the control portion 112-k, 152, 162 and/or the learning portion 114-k, 154, 164. The processor 410 is configured to read from and write to the memory 420. Although the processor 410 is depicted as a respective single component, it may be implemented as respective one or more separate processing components. Similarly, although the memory 420 is depicted as a respective single component, it may be implemented as respective one or more separate components, some or all of which may be integrated/removable and/or may provide permanent / semi-permanent/ dynamic/cached storage.

The computer program code 425 may comprise computer-executable instructions that implement at least some aspects of operation of the control portion 112-k, 152, 162 and/or the learning portion 114-k, 154, 164 when loaded into the processor 410. As an example, the computer program code 425 may include a computer program consisting of one or more sequences of one or more instructions. The processor 410 is able to load and execute the computer program by reading the one or more sequences of one or more instructions included therein from the memory 420. The one or more sequences of one or more instructions may be configured to, when executed by the processor 410, cause the apparatus 400 to perform at least some aspects of operation of the control portion 112-k, 152, 162 and/or the learning portion 114-k, 154, 164. Hence, the apparatus 400 may comprise at least one processor 410 and at least one memory 420 including the computer program code 425 for one or more programs, the at least one memory 420 and the computer program code 425 configured to, with the at least one processor 410, cause the apparatus 400 to perform at least some aspects of operation of the control portion 112-k, 152, 162 and/or the learning portion 114-k, 154, 164.

The computer program code 425 may be provided e.g. a computer program product comprising at least one computer-readable non-transitory medium having the computer program code 425 stored thereon, which computer program code 425, when executed by the processor 410 causes the apparatus 400 to perform at least some aspects of operation of the control portion 112-k, 152, 162 and/or the learning portion 114-k, 154, 164. The computer-readable non-transitory medium may comprise a memory device, a record medium or another article of manufacture that tangibly embodies the computer program. As another example, the computer program may be provided as a signal configured to reliably transfer the computer program.

Reference(s) to a processor herein should not be understood to encompass only programmable processors, but also dedicated circuits such as field-programmable gate arrays (FPGA), application specific circuits (ASIC), signal processors, etc. Features described in the preceding description may be used in combinations other than the combinations explicitly described.

## Claims

1. An apparatus (110-k, 150, 160) for monitoring air quality in a space:
a control portion (112-k, 152, 162) arranged to
obtain sensor data that is at least indirectly descriptive of a concentration of a predefined substance in air within said space,
apply at least one prediction model to predict, based on occurrences of one or more predefined patterns in said sensor data that are associated with respective changes in the concentration of the predefined substance in the air within said space, timing of the concentration of the predefined substance in the air within said space reaching a predefined threshold level due to one of one or more processes that result in introduction of said predefined substance into the air within said space, and
issue, based on the predicted timing, at least one indication that pertains to applicability of said space for human occupants,
**characterized in that** said one or more predefined patterns comprise one or more predefined onset patterns and/or one or more predefined ending patterns, each associated, respectively, with a beginning or an ending of a respective one of said one or more processes and said at least one prediction model comprises a prediction model that is applicable for predicting a time period that is indicative of time from detecting an occurrence of one of said predefined onset patterns or ending patterns until the concentration of said predefined substance in the air within said space reaching the predefined threshold level,
wherein said at least indication comprises one of the following: an indication of a predicted timing of the space becoming non-applicable for human occupants or an indication of a predicted timing of the space becoming applicable for human occupants.

2. An apparatus (110-k) according to claim 1, further comprising a sensor portion (111-k) arranged to capture said sensor data in said space;

3. An apparatus (110-k, 150, 160) according to claim 1 or 2, wherein the control portion (112-k, 152, 162) is arranged to:
apply the first prediction model to detect an occurrence of one of said predefined onset patterns and predict the time from the occurrence of the detected one of said predefined onset patterns until the concentration of said predefined substance in the air within said space exceeding the first predefined threshold level; and/or
apply the second prediction model to detect an occurrence of one of said predefined ending patterns and predict the time from the occurrence of the detected one of said predefined ending patterns until the concentration of said predefined substance in the air within said space falling below the predefined threshold level.

4. An apparatus (110-k, 150, 160) according to any of claims 1 to 3, wherein said indication comprises at least one of the following:
- a visual indication concerning one of applicability or non-applicability of the space for human occupants,
a signal transmitted to another apparatus, where said signal conveys an indication concerning one of applicability or non-applicability of the space for human occupants.

5. An apparatus (110-k, 150, 160) according to any of claims 1 to 4, wherein each of said one or more predefined patterns is associated with a respective one of said one or more processes and wherein the control portion (112-k, 152, 162) is arranged to:
apply said at least one prediction model to identify, based on a detected occurrence of one of said one or more predefined patterns in said sensor data, one of said one or more processes, and
issue at least one indication that pertains to the identified one of said one or more processes.

6. An apparatus (110-k, 150, 160) according to any of claims 1 to 5, further comprising a learning portion (114-k, 154, 164) arranged to:
obtain history data comprising respective histories of the sensor data and concentrations of said predefined substance in the air within said space for a learning period;
carry out a learning procedure to derive, based on the history data, said at least one prediction model.

7. An apparatus (110-k) according to claim 6, wherein the learning portion (114-k) is arranged to:
obtain the history of sensor data via operation of a sensor portion (111-k) of the apparatus (110-k) during the learning period; and
obtain the history of concentrations of said predefined substance in the air within said space via operation of a further sensor coupled to the apparatus (110-k) at least for the duration of the learning period.

8. An apparatus (110-k, 150, 160) according to claim 6 or 7, wherein the learning portion (114-k, 154, 164) is arranged to carry out the learning procedure to determine, based on the history data,
said one or more patterns in sensor data that are associated with respective changes in the concentration of the predefined substance in the air within said space, and
said at least one prediction model that is applicable for predicting the timing of the predefined substance in the air within said space reaching the predefined threshold level based on occurrences of said one or more patterns.

9. An apparatus (110-k, 150, 160) according to any of claims 6 to 8, wherein the history data further comprises a history of indications of respective timings of beginnings and endings of said one or more processes that result in introduction of said predefined substance in the air within said space, said indications obtained via one or more of the following:
in respective control signals received from respective apparatuses involved in carrying out respective ones of said one or more processes,
as user input via a user interface of the apparatus.

10. An apparatus (110-k, 150, 160) according to any of claims 1 to 9, wherein said at least one prediction model comprises one of the following.
an artificial neural network, ANN, arranged to determine the timing of the concentration of the predefined substance in the air within said space reaching the predefined threshold level based on occurrences of said one or more patterns,
a statistical model arranged to determine the timing of the concentration of the predefined substance in the air within said space reaching the predefined threshold level based on occurrences of said one or more patterns.

11. An apparatus (110-k, 150, 160) according to any of claims 1 to 10, wherein said predefined substance comprises a predefined indoor air pollutant.

12. An apparatus (110-k) according to any of claims 1 to 11, wherein
said sensor data comprises respective time series of sensor values captured by respective one or more sensors of a sensor portion (111-k) of the apparatus (110-k), and
each of said one or more predefined patterns include respective sensor values captured by the respective one or more sensors over a monitoring period.

13. An apparatus (110-k, 150, 160) according to any of claims 1 to 12, wherein said sensor data comprises respective sensor values that are indicative of one or more of the following:
total concentration of volatile organic compounds, tVOC, in said space, occupancy in said space;

14. A method (200) for monitoring air quality in a space, the method (200) comprising:
obtaining (202) sensor data that is at least indirectly descriptive of a concentration of a predefined substance in air within said space,
applying (204) at least one prediction model to predict, based on occurrences of one or more predefined patterns in said sensor data that are associated with respective changes in the concentration of the predefined substance in the air within said space, timing of the concentration of the predefined substance in the air within said space reaching a predefined threshold level due to one of one or more processes that result in introduction of said predefined substance into the air within said space; and
issuing (206), based on the predicted timing, at least one indication that pertains to applicability of said space for human occupants,
**characterized in that** said one or more predefined patterns comprise one or more predefined onset patterns and/or one or more predefined ending patterns, each associated, respectively, with a beginning or an ending of a respective one of said one or more processes and said at least one prediction model comprises a prediction model that is applicable for predicting a time period that is indicative of time from detecting an occurrence of one of said predefined onset patterns or ending patterns until the concentration of said predefined substance in the air within said space reaching the predefined threshold level,
wherein said at least indication comprises one of the following: an indication of a predicted timing of the space becoming non-applicable for human occupants or an indication of a predicted timing of the space becoming applicable for human occupants.

15. A method (200, 300) according to claim 14, further comprising deriving said at least one prediction model via carrying out the following:
obtaining (302) history data comprising a history of sensor data that is at least indirectly descriptive of the concentration of the predefined substance in the air within said space for a learning period and a history of concentrations of said predefined substance in the air within said space for the learning period; and
carrying out (304) a learning procedure to derive, based on the history data, at least one prediction model that is applicable for predicting, based on occurrences of one or more patterns in the sensor data that are associated with respective changes in the concentration of the predefined substance in the air within said space, timing of the concentration of the predefined substance in the air within said space reaching the predefined threshold level due to the one of the one or more processes that result in introduction of the predefined substance into the air within said space.
